# EUROPEAN PATENT SPECIFICATION

(11) **EP 2 101 719 B1**
(45) Date of publication and mention of the grant of the patent: **20.02.2019**
(21) Application number: 07851340.5
(22) Date of filing: 07.12.2007
(51) Int. Cl.: A61K 8/02, A61K 8/22, A61K 8/24, A61K 8/25, A61K 8/64, A61K 8/81, A61K 8/86, A61K 8/88, A61Q 11/00

(54) **TEETH WHITENING FUNCTIONAL MATERIALS DELIVERY SYSTEM**
SYSTEM ZUR ABGABE VON FUNKTIONALEN ZAHNBLEICHSTOFFEN
SYSTÈME DE DÉLIVRANCE DE MATÉRIAUX FONCTIONNELS DE BLANCHIMENT DES DENTS

(30) Priority: 07.12.2006 KR 20060124021
(43) Date of publication of application: 23.09.2009
(73) Proprietor: Seoul National University Industry Foundation, Gwanak-gu Seoul 151-050 (KR); Nano Intelligent Biomedical Engineering Corporation Co., Ltd., Seoul 110-749 (KR)
(72) Inventor: CHUNG, Chong-Pyoung, Seoul 138-170 (KR); PARK, Yoon-Jeong, Seoul 152-770 (KR); LEE, Jue-Yeon, Gyeonggi-do 427-736 (KR); PARK, Hyun Jung, Seoul 132-032 (KR)
(74) Representative: Beckmann, Claus
(86) International application number: PCT/KR2007/006369
(87) International publication number: WO 2008/069622

(56) References cited:
- WO-A1-2005/058266
- GB-A- 2 354 441
- KR-A- 20060 102 564
- US-A1- 2003 072 722
- US-A1- 2004 105 834
- US-A1- 2004 241 110
- US-B1- 6 221 341

## Description

### TECHNICAL FIELD

The present invention relates to a teeth whitening functional agent delivery system, and more particularly, to a tooth whitening composition comprising an adhesive polymer, a tooth whitening active material, a material for promoting penetration of the tooth whitening active material (a penetration enhancer), a surfactant, a stabilizer, an antioxidant, an antihypersensitive agent, pyrophosphate and SiO₂.

### BACKGROUND ART

Teeth consist of an inner layer of dentin and an outer layer of enamel which serves as a protective wall. The enamel layer of the tooth is naturally an opaque white or slightly off-white color. The enamel layer has microscopic spaces or pores, into which discoloring materials or discolorants penetrate to discolor teeth.

Many materials that individuals come into contact with daily can stain teeth or cause off-white teeth. Particularly, food and tobacco can be accumulated on the tooth's enamel layer to form a thin film on tooth enamel, and these discoloring materials can pass through the enamel layer. While this phenomenon is repeated for a long time, teeth can be discolored. Also, diseases or environmental factors can influence the discoloration of teeth. Although discolored teeth do not cause health problems, the desire for bright white teeth is continuously increasing.

Among tooth whitening agents which are currently used, fluorine-containing toothpastes are used to chemically remove staining substances such as foods, nicotine, coffee and tea, adhered to the tooth surface, or tooth stains caused by accumulation of tartar deposits on the tooth surface, and are generally used to make the mouth feel fresh or reduce halitosis. However, it was difficult to achieve tooth whitening effect through tooth brushing with the toothpastes.

Thus, methods for in-clinic teeth whitening or at-home teeth whitening, which are professional tooth whitening programs, were developed. The whitening treatment in dental clinics starts with preparing a rubber dam, which is fitted into the mouth in order to prevent hydrogen peroxide whitening agent coming into contact with oral soft tissue.

The at-home teeth whitening program is different from the in-clinic teeth whitening program, in that patients themselves bleach the teeth with a low strength whitening agent a few hours per day over the course of a few weeks. In at-home teeth whitening program, it is needed that a specific device fit into the patient's mouth in dental clinic at the initial fitting. The device is manufactured so as to fit into the patient's teeth, such that whitening gel is delivered to the patient's teeth. However, when using the above-described methods, much cost is incurred, and in addition, it cause difficulties in daily life.

US Patent 5,425,953 discloses a liquid polymer composition for tooth bleaching. The liquid polymer composition was developed to overcome the shortcomings of gel products using a two-part system which is in the liquid state before application to the teeth and is changed to a solid film after application to the teeth. However, the liquid polymer composition has problems in that it is sticky for a given time during which a film is produced after application to the teeth and in that it is harmful to the human body because of the use of organic solvent.

US Patent 5,891,453 discloses a delivery system for a tooth whitener using a strip of material having low flexural stiffness. The disclosed whitener delivery system does not make daily life inconvenient, but is inconvenient in that the strip material, that is, the waterproof strip material, should be detached after a given time period of application to the teeth. Also, the tooth whitening strip is easily attached to the teeth through the control of the flexural stiffness thereof, but has problems in that the waterproof film itself has a foreign body sensation, and because the existing whitening gel is simply applied to the film, it adheres to hands or is highly sticky, and thus it is not easy to deliver the whitening active material.

US2004/105834A1 discloses teeth whitening compositions and GB2354441A discloses compositions for treating dentine hypersensitivity.

Therefore, in the art, there is a need to develop a tooth whitening composition, which maintains the sensory feel of the existing tooth whitening material, shows an increased penetration of tooth whitening agents into the stained pores of the teeth, and is not eroded for a period of time sufficient to whiten tooth enamel, so that it can maintain the tooth whitening effect for a long period of time.
Accordingly, the present inventors have made extensive efforts to develop a tooth whitening composition, which shows an increased penetration of tooth whitening agents into the stained pores of the teeth and an excellent tooth whitening effect, compared to those of the prior tooth whitening products. As a result, the present inventors have found that a tooth whitening composition comprising an adhesive polymer, a tooth whitening active material, a material for promoting penetration of the tooth whitening active material, a hydrophobic surfactant, a stabilizer, pyrophosphate and SiO₂, quickly shows a tooth whitening effect and maintains the effect for a long period of time, while it maintains the sensory feel of the prior tooth whitening products, thereby completing the present invention.

### SUMMARY OF THE INVENTION

It is an object of the present invention to provide a tooth whitening composition, which shows an increased penetration of tooth whitening agents into the stained pores of the teeth and an excellent tooth whitening effect, and quickly shows a tooth whitening effect to maintain the effect for a long period of time.

To achieve the above objects, the present invention provides a tooth whitening composition according to claim 1.

Other features and aspects of the present invention will be apparent from the following detailed description and the appended claims.

### BRIEF DESCRIPTION OF DRAWINGS

FIG. 1 is a graphic diagram showing analysis results for the release of hydrogen peroxide according to the kind of a surfactant in the tooth whitening composition according to the present invention.

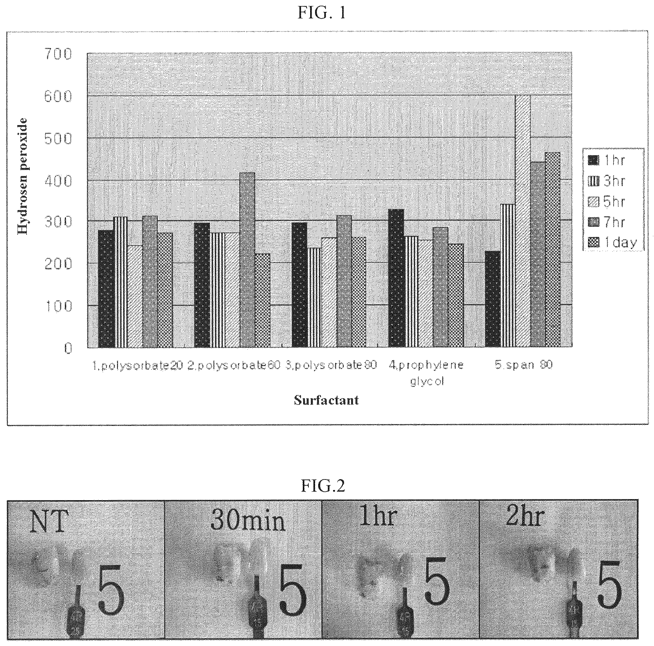

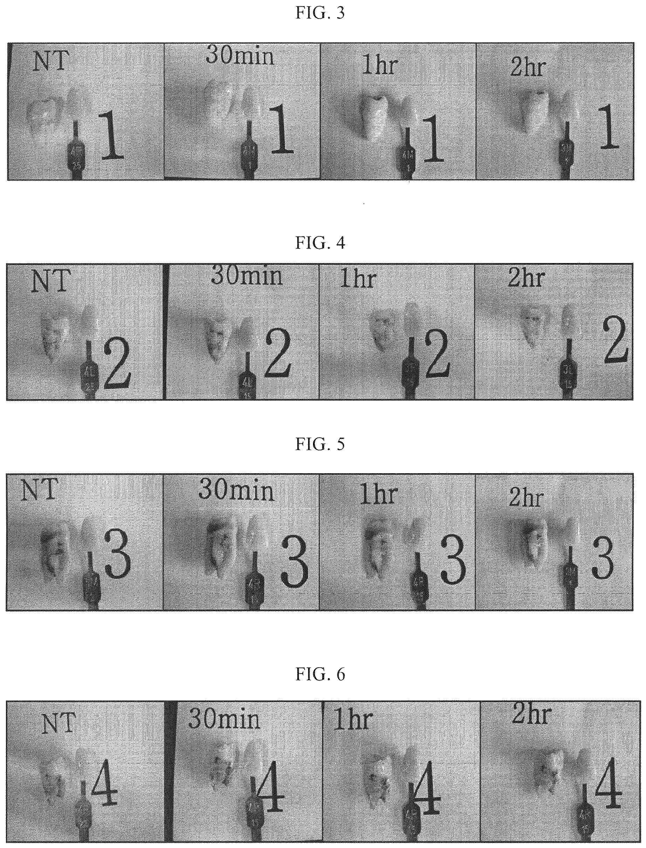

FIGS. 2 to 6 are photographs showing the change in color of non-vital teeth according to the content of a material for promoting penetration of a tooth whitening active material (FIG. 2: a group treated with a gel without material for promoting penetration of the tooth whitening active material; FIG. 3: a group treated with a gel containing 0.5 wt% of the material for promoting penetration of the tooth whitening active material; FIG. 4: a group treated with a gel containing 1 wt% of the material for promoting penetration of the tooth whitening active material; FIG. 5: a group treated with a gel containing 2 wt% of the material for promoting penetration of the tooth whitening active material; and FIG. 6: a group treated with a gel containing 5 wt% of the material for promoting penetration of the tooth whitening active material).
FIG. 7 is a graphic diagram numerically showing the degree of the change in color of the teeth according to the content of the penetration enhancer for tooth whitening active material in a tooth whitening agent delivery system.

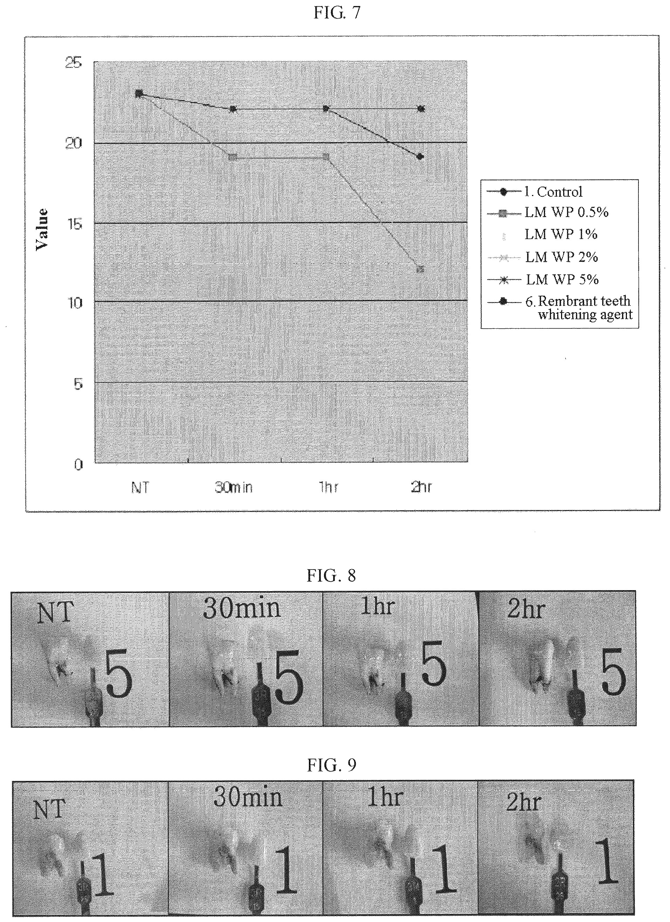

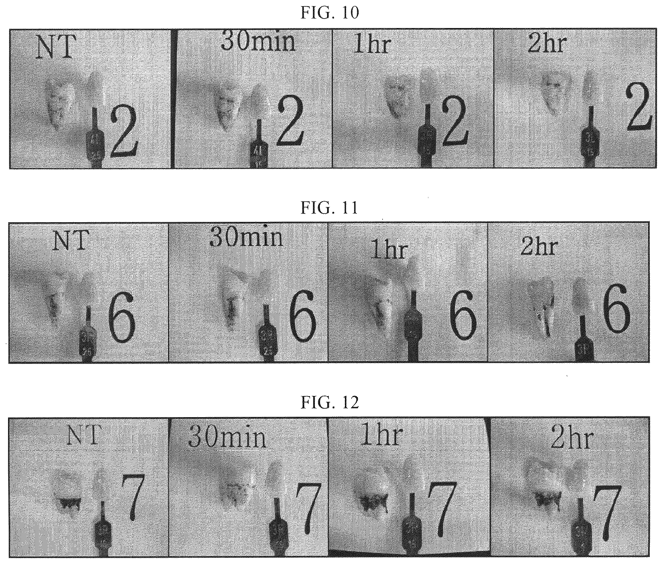

FIGS. 8 to 12 are photographs showing the change in color of non-vital teeth according to the contents of the penetration enhancer for tooth whitening active material and SiO₂ (FIG. 8: a group treated with a gel without the penetration enhancer for tooth whitening active material and SiO₂; FIG. 9: a group treated with a gel containing 0.5 wt% of the penetration enhancer for tooth whitening active material and 0.5 wt% of SiO₂; FIG. 10: a group treated with a gel containing 0.8 wt% of the penetration enhancer for tooth whitening active material and 0.8 wt% of SiO₂; FIG. 11: a group treated with a gel containing 0.5 wt% of SiO₂; and FIG. 12: a group treated with a gel containing 0.8 wt% of SiO₂).
FIG. 13 is a graphic diagram showing the change in color of teeth according to the contents of the penetration enhancer for tooth whitener and SiO₂.

### DETAILED DESCRIPTION OF THE INVENTION, AND PREFERRED EMBODIMENTS

The present invention relates to a tooth whitening composition according to claim 1, which has an excellent penetration into the stained pores of the teeth and an excellent tooth whitening effect, and quickly shows a tooth whitening effect to maintain the effect for a long period of time.

In the present invention, the adhesive polymer is one or more than polymers selected from the group consisting of hydroxypropylmethylcellulose, hydroxyethylcellulose, polyvinyl alcohol, polyvinyl pyrrolidone, hydroxypropylcellulose, carbomer (polyacrylic acid derivative), a copolymer of methyl vinyl ether and maleic anhydride, and polyethylene oxide.
The adhesive polymer serves as a tooth adhesive in the tooth whitening composition according to the present invention. Upon hydration, it is preferably contained in an amount of 5-80 parts by weight, and more preferably 5-60 parts by weight, based on 100 parts by weight of the inventive tooth whitening composition. If the content of the adhesive polymer in the tooth whitening composition is less than 5 parts by weight, the viscosity of the whitening composition layer will be too low, so that it will run off, and thus will be difficult to apply it to the teeth upon treatment. On the other hand, if the content of the adhesive polymer is more than 80 parts by weight, the viscosity of the tooth whitening composition layer will be too high, thus making the treatment difficult.

The polymer showing adhesion to the teeth upon hydration is preferably polyethylene oxide having a weight-average molecular weight of 100,000-4,000,000, and more preferably a polyethylene oxide having a weight-average molecular weight of 100,000-2,000,000. If the molecular weight of the polyethylene oxide is less than 100,000, it will be difficult to reach a viscosity suitable for application in conventional use, and if it is more than 4,000,000, it will have high viscosity even at low concentration, thus making the application thereof difficult.

In the present invention, the tooth whitening active material is one or more than two substances selected from the group consisting of peroxide, metal chlorite, perborate, percarbonate and peroxy acid. The peroxide is preferably one or more than two substances selected from the group consisting of hydrogen peroxide, calcium peroxide and carbamide peroxide, and the metal chlorite is preferably one or more than two substances selected form the group consisting of calcium chlorite, barium chlorite, magnesium chlorite, lithium chlorite, sodium chlorite and potassium chlorite.
The tooth whitening active material is preferably contained in an amount of 0.1-50 parts by weight, and more preferably 0.5-30 parts by weight, based on 100 parts by weight of the tooth whitening composition. If the content of the tooth whitening active material is less than 0.1, the desired whitening effect cannot be obtained, and if it exceeds 50 parts by weight, it will have safety problems, including tooth erosion and oral mucosa damage.

In the present invention, the material for promoting penetration of the tooth whitening active material (a penetration enhancer) is a peptide having an arginine content of more than 80%. The peptide having an arginine content of more than 80% is one or more than two substances selected from protamine fragments. The peptide having an arginine content of more than 80%, which is the material for promoting penetration of the tooth whitening active material, is preferably contained 0.1-50 parts by weight, and more preferably 0.5-20 parts by weight, based on 100 parts by weight of the tooth whitening composition. If the content thereof is less than 0.1 parts by weight, the desired penetration effect cannot be obtained, and if it exceeds 50 parts by weight, it will interfere with the penetration of the tooth whitening active material, thus causing safety problems such as oral mucosa irritation.

In the present invention, the surfactant is one or more than two substances selected from the group consisting of polyethylene glycol, propylene glycol, glycerin, triethyl citrate, polysorbate and Span. The hydrophobic surfactant is used to increase the flexural stiffness of the tooth whitening composition and is preferably contained in an amount of 5.0-50 parts by weight based on 100 parts by weight of the composition. If the content thereof is less than 5.0 parts by weight, the flexibility of the composition will be reduced, so that it will give a foreign body sensation when it is used, and if it exceeds 50 parts by weight, the stability of the resulting whitening composition will be reduced.

In the present invention, the stabilizer is preferably one or more than two chelating agents selected from the group consisting of ethylenediaminetetraacetic acid and a salt thereof, sodium stannate and potassium stannate, or one or more than two antioxidants selected from the group consisting of butylated hydroxy toluene, nordihydroguaiaretic acid, proryl gallate, trihydroxybutyrophenone and butylated hydroxyanisol.
The stabilizer is used to enhance the stability of the tooth whitening active material and is preferably contained in an amount of 0.10-5.0 parts by weight based on 100 parts by weight of the whitening composition.
In the present invention, the pyrophosphate is preferably selected from the group consisting of Na₄P₂O₇, K₄P₂O₇, Na₂H₂P₂O₇ and K₂H₂P₂O₇.
The pyrophosphate has the effects of removing tartar and metal-chelating substance and is used in the inventive tooth whitening material delivery system to prevent tartar from forming on the teeth. It is preferably contained in an amount of 1.0-10.0 parts by weight based on 100 parts by weight of the whitening composition.

Also, said SiO₂ is frequently used as a polishing agent to remove dental tartar or plaque adhered to the teeth and to impart original gloss to the tooth surface. It can be used in the form of nanoparticles to increase the whitening effect. The content of SiO₂ in the composition is preferably 0.1-2.0 parts by weight, and more preferably 0.3-1.0 part by weight, based on 100 parts by weight of the composition. If the content thereof is less than 0.1 parts by weight, the effect thereof will be reduced, and if it exceeds 2.0 parts by weight, any significant increase in the effect thereof according to an increase in the amount of addition thereof will not be shown.

The composition according to the present invention may contain, in addition to the above-described components, conventional additives for use in tooth whitening material delivery systems, including a pH adjusting agent, a sweetening agent, a fragrance, and an antihypersensitive agent.

In the present invention, the pH adjusting agent is preferably one or more than two substances selected from the group consisting of sodium phosphate dibasic, sodium phosphate tribasic, sodium pyrophosphate, potassium pyrophosphate, sodium hydrogen carbonate, potassium hydroxide, ammonium hydroxide, triethanolamine, citric acid, sodium citrate and sodium hydroxide. The pH adjusting agent serves to adjust the pH of the inventive tooth whitening composition to 4.0-11.0 for the long-term storage stability of the composition.
The sweetening agent is preferably one or more than tow substances selected from the group consisting of sorbitol, mannitol, xylitol, stevioside and saccharine, and the fragrance is preferably one or more than tow substances selected from the group consisting of menthol, peppermint oil, L-menthol and cinnamic acid. Also, the antihypersensitive agent is preferably one or more than tow substances selected from the group consisting of potassium nitrate, stannous fluoride (SnF₂), sodium monoflouride phosphate (MFP), sodium fluoride (NaF), sodium silicoflouride (Na₂SiF₆) and calcium hydroxide (Ca(OH)₂).

In addition, as a solvent for dissolving said components, ethanol, water and a mixture of ethanol and water (9:1-6:4 % (v/v)) may be used.

### Examples

Hereinafter, the present invention will be described in further detail with reference to examples. It will be apparent to one skilled in the art that these examples are for illustrative purpose only and are not construed to limit the scope of the present invention.

### Example 1: Preparation of tooth whitening composition

### 1-1: Selection of tooth-adhesive polymer and addition of tooth whitening active material

0.4 g of each of adhesive polymers (carbomer 934, carbomer 504, carbomer 505, carbomer 501, carbomer 940 and carbomer 941) (SIMITOMO SEIKA, Japan) (Noveon, USA)) was dissolved in 4 ml of IN NaOH and then left to stand for 24 hours. Then, 3 ml of stabilizer, glycerin and 0.1 ml of hydrophobic surfactant, Span 80 (Sigma chemical, USA) were added thereto.

As a tooth whitening active material, carbamide peroxide was added thereto in an amount of 8.05 wt% based on the total weight of the composition to make a whitening gel. To the gel, 0.1 g of potassium nitrate having an antihypersentitive effect, and sodium citrate having a whitening effect, were added. In order to prevent the decomposition of unstable hydrogen peroxide, sodium stannate and sodium acid pyrophosphate as stabilizers were added thereto. Also, 0.1 g of ethylenedieminetetraacetic acid (EDTA) as an antioxidant was added and 0.1 g of sodium perborate having both an antioxidant effect and a whitening effect was added. To the mixture, fragrance L-mentol (Tien Yuan Chemical, Singapore) was added in an amount of 0.1 wt% based on the total weight of the composition, and the resulting mixture was uniformly blended, thus preparing a basic whitening gel.

### 1-2: Addition of material for promoting penetration of tooth whitening active material

To the composition prepared in Example 1-1, a peptide having an arginine content of more than 80%, as a delivery-promoting material for tooth whitener, capable of significantly increasing penetration of the tooth whitening active material into the stained pores of the teeth, was added in amounts of 0.5 wt%, 1 wt%, 2 wt% and 5 wt% based on the total weight of the whitening gel.

### 1-3: Addition of SiO₂

To the composition prepared in Example 1-2, SiO₂ particles having a size of about 10 nm, which is frequently used as a polishing agent to remove dental tartar or plaque adhered to the teeth and to impart original gloss to the tooth surface, was added, thus preparing the inventive tooth whitening composition.

### Example 2: Selection of surfactant suitable for tooth whitening product

In order for the tooth whitening effect to be maintained for a long period of time by avoiding contact with water and protecting the gel from being eroded by saliva in the mouth, a hydrophobic surfactant was added to the whitening gel.

Specifically, whitening gels containing the same components except for a hydrophobic surfactant were prepared using 0.4 g of adhesive polymer, Carbomer 940 in the same manner as in Example 1. To the whitening gels, each of hydrophobic surfactants Polysorbate 20, Polysorbate 60, Polysorbate 80, propylene glycol and Span 80 was added and the resulting whitening gels. The whitening gels were tested for hydrogen peroxide release.

Specifically, 2 ml of each of the samples was placed in a glass vial, to which 1 ml of distilled water was added. After 1 hour, 0.5 ml of distilled water was additionally added to the glass vial, and then 0.5 ml of each of the samples was transferred into a fresh tube. In order to obtain a standard curve for determining the hydrogen peroxide release, 900 *µ*M H₂0₂ was diluted stepwise, thus preparing standard solutions having concentrations of 0 *µ*M, 150 *µ*M, 300 *µ*M, 450 *µ*M, 600 *µ*M and 900 *µ*M.
Also, 100 *µ*L of 2.5 M potassium nitrate and 200 *µ*L of 10 mM ferrous ammonium sulfate were added to each of the samples. When the color of each solution changed from colorless to red, 200 *µ*L of the standard solution and each sample solution were placed in a 96-well plate, and then measured for absorbance at 467 nm. Using the same method as described above, the absorbance was also measured after 3 hr, 5 hr, 7 hr and 1 day (FIG. 1). FIG. 1 shows the results of the hydrogen peroxide release test of the whitening gels prepared according to the present invention. As shown in FIG. 1, hydrogen peroxide release from the whitening gel containing Span 80 was the highest.

### Example 3: Test of non-vital teeth in according to the addition of various amounts of material for promoting penetration of tooth whitening active material

In order to examine the effect of the material for promoting penetration of the tooth whitening active material, a material for promoting penetration of the tooth whitening active material was added to the whitening gel, prepared in Example 1-1, to concentrations of 0.5 wt%, 1 wt%, 2 wt% and 5 wt% based on the total weight of the whitening gel.

Non-vital teeth used in the experiment were provided by Seoul National University Dental Hospital. To wash the non-vital teeth, the non-vital teeth were placed in a beaker filled with distilled water, and then washed five times in a sonicator, followed by drying.

In order to examine the effect of the material for promoting penetration of the tooth whitening active material, the penetration enhancer was added in amounts of 0.5 wt%, 1 wt%, 2 wt% and 5 wt% based on the total weight of the whitening gel, thus preparing gels.

2 ml of each of the gels was added to each well, into which the enamel portion of the teeth was inserted. Then, the gels were tested for NT, 30 min, 1 hr and 2 hr. The change in the shade of the teeth was measured using Vitapan 3D-Master Shade Guide (VITA Zahnfabrik H.Rauter GmbH & Co. KG, Germany).

FIGS. 2 to 6 are photographs showing the change in color of the non-vital teeth according to the amount of the material for promoting penetration of the tooth whitening active material (FIG. 2: a group treated with a gel without the material for promoting penetration of the tooth whitening active material; FIG. 3: a group treated with a gel containing 0.5 wt% of the material for promoting penetration of the tooth whitening active material; FIG. 4: a group treated with a gel containing 1 wt% of the material for promoting penetration of the tooth whitening active material; FIG. 5: a group treated with a gel containing 2 wt% of the material for promoting penetration of the tooth whitening active material; and FIG. 6: a group treated with a gel containing 5 wt% of the material for promoting penetration of the tooth whitening active material). FIG. 7 graphically shows the change in color of the non-vital teeth according to the amount of the material for promoting penetration of the tooth whitening material.

As a result, as shown in FIGS. 2 to 6 and 7, the group treated with the gel containing 0.5 wt% of the material for promoting penetration of the tooth whitening active material showed the most excellent effect.

### Example 4: Test of non-vital teeth according to the addition of various amounts of SiO₂

In order to measure the tooth whitening effect of SiO₂, which is frequently used as a polishing agent to remove dental tartar or plaque adhered to the teeth and to impart original gloss to the tooth surface, the material for promoting penetration of the tooth whitening active material was added to the whitening gel, prepared in Example 1-1, in amounts of 0.5 wt% and 0.8 wt%, and SiO₂ nanoparticles having a size of about 10 nm were added to the whitening gel in amounts of 0.5 wt% and 0.8 wt%, thus preparing whitening gels. Then, each of the gels, to which both the penetration enhancer for tooth whitening active material and SiO₂ were not added, and the gels containing 0.5 wt% of SiO₂ or 0.8 wt% of SiO₂, was used to test non-vital teeth. The non-vital teeth were used in the experiment, after they were washed clean and dried in the same manner as in Example 3.

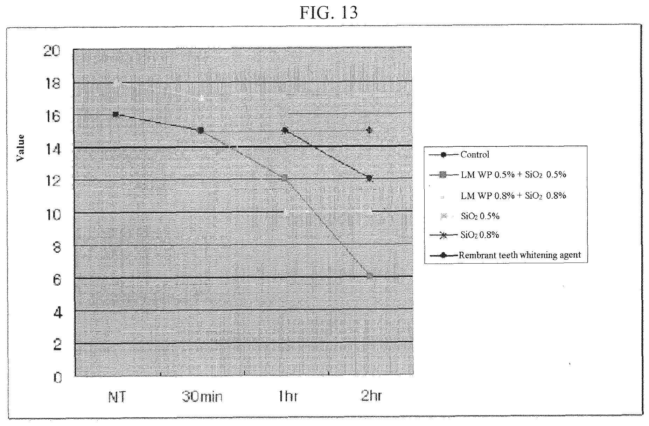

FIGS. 8 to 12 are photographs showing the change in color of the non-vital teeth according to the contents of the penetration enhancer for tooth whitening active material and SiO₂ (FIG. 8: a group treated with a gel without the penetration enhancer for tooth whitening active material and SiO₂; FIG. 9: a group treated with a gel containing 0.5 wt% of the penetration enhancer for tooth whitening active material and 0.5 wt% of SiO₂; FIG. 10: a group treated with a gel containing 0.8 wt% of the penetration enhancer for tooth whitening active material and 0.8 wt% of SiO₂; FIG. 11: a group treated with a gel containing 0.5 wt% of SiO₂; and FIG. 12: a group treated with a gel containing 0.8 wt% of SiO₂). FIG. 13 graphically shows the change in color of the non-vital teeth according to the contents of the penetration enhancer for tooth whitening active material and SiO₂.

As a result, as shown in FIGS. 8 to 12 and 13, the group treated with the gel containing 0.5 wt% of the penetration enhancer for tooth whitening active material and 0.5 wt% of SiO₂ showed the most excellent effect.

### INDUSTRIAL APPLICABILITY

As described and proved in detail above, the present invention provides a tooth whitening composition, which shows an increased penetration rate of a whitening active material and has an excellent whitening effect, compared to the prior tooth whitening products. The tooth whitening composition according to the present invention has advantages in that it maintains the sensory feel of the prior tooth whitening products, while it quickly shows a tooth whitening effect which is to maintain the effect for a long period of time.

## Claims

1. A tooth whitening composition comprising:
an adhesive polymer; a tooth whitening active material; a peptide having an arginine content of more than 80%, selected from protamine fragments as a material for promoting penetration of the tooth whitening active material; a surfactant; a stabilizer; an antioxidant; an antihypersensitive agent; pyrophosphate; and SiO₂,
wherein the adhesive polymer is one or more selected from the group consisting of hydroxypropylmethylcellulose, hydroxyethylcellulose, polyvinyl alcohol, polyvinyl pyrrolidone, hydroxypropylcellulose, carbomer (polyacrylic acid derivative), a copolymer of methyl vinyl ether and maleic anhydride, and polyethylene oxide,
wherein the tooth whitening active material is one or more selected from the group consisting of peroxide, metal chlorite, perborate, percarbonate and peroxy acid,
wherein the surfactant is one or more selected from the group consisting of polyethylene glycol, propylene glycol, glycerin, triethyl citrate, polysorbate and Span.

2. The tooth whitening composition according to claim 1, wherein the adhesive polymer is polyethylene oxide having a weight-average molecular weight of 100,000-4,000,000.

3. The tooth whitening composition according to claim 1, wherein said peroxide is one or more selected from the group consisting of hydrogen peroxide, calcium peroxide and carbamide peroxide.

4. The tooth whitening composition according to claim 1, wherein said metal chlorite is one or more selected form the group consisting of calcium chlorite, barium chlorite, magnesium chlorite, lithium chlorite, sodium chlorite and potassium chlorite.

5. The tooth whitening composition according to claim 1, wherein the stabilizer is one or more chelating agents selected from the group consisting of ethylenediaminetetraacetic acid and a salt thereof, sodium stannate and potassium stannate.

6. The tooth whitening composition according to claim 1, wherein the antioxidant is one or more antioxidants selected from the group consisting of butylated hydroxy toluene, nordihydroguaiaretic acid, proryl gallate, trihydroxybutyrophenone and butylated hydroxyanisol.

7. The tooth whitening composition according to claim 1, wherein the pyrophosphate is selected from the group consisting of Na₄P₂O₇, K₄P₂O₇, Na₂H₂P₂O₇ and K₂H₂P₂O₇.

8. The tooth whitening composition according to claim 1, which contains: the adhesive polymer 5-80 parts by weight; the tooth whitening active material 1-50 parts by weight; the material for promoting penetration of the tooth whitening active material 0.1-50 parts by weight; the surfactant 5-50 parts by weight; the stabilizer 0.10-5.0 parts by weight; pyrophosphate 1-10 parts by weight; and SiO₂-0.1-2.0 parts by weight, based on 100 parts by weight of the tooth whitening composition.

9. The tooth whitening composition according to claim 1, which additionally comprises one or more selected from the group consisting of a pH adjusting agent, a sweetening agent, and a fragrance.

10. The tooth whitening composition according to claim 9, wherein the pH adjusting agent is one or more selected from the group consisting of sodium phosphate dibasic, sodium phosphate tribasic, sodium pyrophosphate, potassium pyrophosphate, sodium hydrogen carbonate, potassium hydroxide, ammonium hydroxide, triethanolamine, citric acid, sodium citrate and sodium hydroxide.

11. The tooth whitening composition according to claim 9, wherein the pH of the composition is 4.0-11.0.

## Patentansprüche

1. Zahnbleichzusammensetzung, umfassend:
ein adhäsives Polymer; ein Zahnbleich-Aktivmaterial; ein Peptid, das einen Arginingehalt von mehr als 80% hat, das aus Protaminfragmenten ausgewählt ist, als Material zur Förderung der Penetration des Zahnbleich-Aktivmaterials; ein Surfactant; einen Stabilisator; ein Antioxidans; ein Mittel gegen Hypersensibilität; Pyrophosphat und SiO₂,
wobei das adhäsive Polymer eins oder mehrere, ausgewählt aus der Gruppe, bestehend aus Hydroxypropylmethylcellulose, Hydroxyethylcellulose, Polyvinylalkohol, Polyvinylpyrrolidon, Hydroxypropylcellulose, Carbomer (Polyacrylsäurederivat), einem Copolymer von Methylvinylether und Maleinsäureanhydrid und Polyethylenoxid, ist,
wobei das Zahnbleich-Aktivmaterial eins oder mehrere, ausgewählt aus der Gruppe, bestehend aus Peroxid, Metallchlorit, Perborat, Percarbonat und Peroxysäure, ist,
wobei das Surfactant eins oder mehrere, ausgewählt aus der Gruppe, bestehend aus Polyethylenglycol, Propylenglycol, Glycerin, Triethylcitrat, Polysorbat und Span, ist.

2. Zahnbleichzusammensetzung gemäß Anspruch 1, wobei das adhäsive Polymer Polyethylenoxid mit einem gewichtsmittleren Molekulargewicht von 100.000-4.000.000 ist.

3. Zahnbleichzusammensetzung gemäß Anspruch 1, wobei das Peroxid eins oder mehrere, ausgewählt aus der Gruppe, bestehend aus Wasserstoffperoxid, Calciumperoxid und Carbamidperoxid, ist.

4. Zahnbleichzusammensetzung gemäß Anspruch 1, wobei das Metallchlorit eins oder mehrere, ausgewählt aus der Gruppe, bestehend aus Calciumchlorit, Bariumchlorit, Magnesiumchlorit, Lithiumchlorit, Natriumchlorit und Kaliumchlorit, ist.

5. Zahnbleichzusammensetzung gemäß Anspruch 1, wobei der Stabilisator ein oder mehrere Chelatbildner, ausgewählt aus der Gruppe, bestehend aus Ethylendiamintetraessigsäure und einem Salz davon, Natriumstannat und Kaliumstannat, ist.

6. Zahnbleichzusammensetzung gemäß Anspruch 1, wobei das Antioxidans ein Antioxidans oder mehrere Antioxidantien, ausgewählt aus der Gruppe, bestehend aus butyliertem Hydroxytoluol, Nordihydroguaiaretsäure, Prorylgalat, Trihydroxybutyrophenon und butyliertem Hydroxyanisol, ist.

7. Zahnbleichzusammensetzung gemäß Anspruch 1, wobei das Pyrophosphat ausgewählt ist aus der Gruppe, bestehend aus Na₄P₂O₇, K₄P₂O₇, Na₂H₂P₂O₇ und K₂H₂P₂O₇.

8. Zahnbleichzusammensetzung gemäß Anspruch 1, welche enthält: das adhäsive Polymer mit 5-80 Gewichtsteilen; das Zahnbleich-Aktivmaterial mit 1-50 Gewichtsteilen; das Material zur Begünstigung der Penetration des Zahnbleich-Aktivmaterials mit 0,1-50 Gewichtsteilen; das Surfactant mit 5-50 Gewichtsteilen; den Stabilisator mit 0,10-5,0 Gewichtsteilen; Pyrophosphat mit 1-10 Gewichtsteilen und SiO₂ mit 0,1-2,0 Gewichtsteilen, bezogen auf 100 Gewichtsteile der Zahnbleichzusammensetzung.

9. Zahnbleichzusammensetzung gemäß Anspruch 1, die außerdem eins oder mehrere, ausgewählt aus der Gruppe, bestehend aus einem Mittel zur Einstellung des pH, einem Süßungsmittel und einem Aromamittel, umfasst.

10. Zahnbleichzusammensetzung gemäß Anspruch 9, wobei das Mittel zur Einstellung des pH eins oder mehrere, ausgewählt aus der Gruppe, bestehend aus dibasischem Natriumphosphat, tribasischem Natriumphosphat, Natriumpyrophosphat, Kaliumpyrophosphat, Natriumhydrogencarbonat, Kaliumhydroxid, Ammoniumhydroxid, Triethanolamin, Citronensäure, Natriumcitrat und Natriumhydroxid, ist.

11. Zahnbleichzusammensetzung gemäß Anspruch 9, wobei der pH der Zusammensetzung 4,0-11,0 ist.

## Revendications

1. Composition de blanchiment de dents comprenant :
un polymère adhésif ; un matériau actif de blanchiment de dents ; un peptide ayant une teneur en arginine de plus de 80 %, choisi parmi des fragments de protamine comme matériau pour promouvoir la pénétration du matériau actif de blanchiment de dents ; un tensioactif ; un stabilisant ; un antioxydant ; un agent anti-hypersensibilité ; un pyrophosphate ; et du SiO₂,
dans laquelle le polymère adhésif est un ou plusieurs choisi(s) dans le groupe constitué de l'hydroxypropylméthylcellulose, de l'hydroxyéthylcellulose, de l'alcool polyvinylique, de la polyvinylpyrrolidone, de l'hydroxypropylcellulose, d'un carbomère (dérivé d'acide polyacrylique), d'un copolymère d'éther méthylvinylique et d'anhydride maléique et d'un poly(oxyde d'éthylène),
dans laquelle le matériau actif de blanchiment de dents est un ou plusieurs choisis dans le groupe constitué d'un peroxyde, d'un chlorite de métal, d'un perborate, d'un percarbonate et d'un peroxyacide,
dans laquelle le tensioactif est un ou plusieurs choisi(s) dans le groupe constitué d'un polyéthylèneglycol, d'un propylèneglycol, de glycérine, de citrate de triéthyle, de polysorbate et de Span.

2. Composition de blanchiment de dents selon la revendication 1, dans laquelle le polymère adhésif est du poly(oxyde d'éthylène) ayant un poids moléculaire moyen en poids de 100 000 à 4 000 000.

3. Composition de blanchiment de dents selon la revendication 1, dans laquelle ledit peroxyde est un ou plusieurs choisi(s) dans le groupe constitué du peroxyde d'hydrogène, du peroxyde de calcium et du peroxyde de carbamide.

4. Composition de blanchiment de dents selon la revendication 1, dans laquelle ledit chlorite de métal est un ou plusieurs corps choisis dans le groupe constitué du chlorite de calcium, du chlorite de baryum, du chlorite de magnésium, du chlorite de lithium, du chlorite de sodium et du chlorite de potassium.

5. Composition de blanchiment de dents selon la revendication 1, dans laquelle le stabilisant est un ou plusieurs agents chélatants choisis dans le groupe constitué de l'acide éthylènediaminetétraacétique et d'un de ses sels, du stannate de sodium et du stannate de potassium.

6. Composition de blanchiment de dents selon la revendication 1, dans laquelle l'antioxydant est un ou plusieurs antioxydants choisis dans le groupe constitué de l'hydroxytoluène butylé, de l'acide nordihydroguaiarétique, du gallate de propyle, de la trihydroxybutyrophénone et de l'hydroxyanisol butylé.

7. Composition de blanchiment de dents selon la revendication 1, dans laquelle le pyrophosphate est choisi dans le groupe constitué de Na₄P₂O₇, K₄P₂O₇, Na₂H₂P₂O₇ et K₂H₂P₂O₇.

8. Composition de blanchiment de dents selon la revendication 1, qui contient 5 à 80 parties en poids de polymère adhésif ; 1 à 50 parties en poids de matériau actif de blanchiment de dents ; 0,1 à 50 parties en poids de matériau de promotion de la pénétration du matériau actif de blanchiment de dents ; 5 à 50 parties en poids de tensioactif ; 0,10 à 5,0 parties en poids de stabilisant ; 1 à 10 parties en poids de pyrophosphate ; et 0,1 à 2,0 parties en poids de SiO₂ sur la base de 100 parties en poids de la composition de blanchiment de dents.

9. Composition de blanchiment de dents selon la revendication 1, qui comprend en outre un ou plusieurs choisi(s) dans le groupe constitué d'un agent de réglage de pH, d'un agent adoucissant et d'un parfum.

10. Composition de blanchiment de dents selon la revendication 9, dans laquelle l'agent de réglage de pH est un ou plusieurs choisi(s) dans le groupe constitué de phosphate de sodium dibasique, de phosphate de sodium tribasique, de pyrophosphate de sodium, de pyrophosphate de potassium, d'hydrogénocarbonate de sodium, d'hydroxyde de potassium, d'hydroxyde d'ammonium, de triéthanolamine, d'acide citrique, de citrate de sodium et d'hydroxyde de sodium.

11. Composition de blanchiment de dents selon la revendication 9, dans laquelle le pH de la composition est de 4,0 à 11,0.
